# EUROPEAN PATENT APPLICATION

(11) **EP 0 969 086 A1**
(43) Date of publication of application: **05.01.2000**
(21) Application number: 97929493.1
(22) Date of filing: 02.07.1997
(51) Int. Cl.: C12N 1/20, C12P 7/64

(54) **MICROORGANISMS PRODUCING DOCOSAHEXAENOIC ACID AND PROCESS FOR THE PRODUCTION OF DOCOSAHEXAENOIC ACID**

(30) Priority: 03.07.1996 JP 17355096; 13.11.1996 JP 30138096; 22.04.1997 JP 10408397
(71) Applicant: SAGAMI CHEMICAL RESEARCH CENTER, Sagamihara-shi, Kanagawa 229 (JP); FUJIYAKUHIN COMPANY, LIMITED, Omiya-shi, Saitawa 331 (JP)
(72) Inventor: YAZAWA, Kazunaga, Fujisawa-shi, Kanagawa 251 (JP); WATANABE, Kazuo, Sagamihara-shi, Kanagawa 228 (JP); ISHIKAWA, Chikako, Tokyo 157 (JP); TOMITA, Miho, Yokohama-shi, Kanagawa 226 (JP); MURAMATSU, Hiroshi, Hino-shi, Tokyo 191 (JP)
(74) Representative: Weisert, Annekäte, Dipl.-Ing. Dr.-Ing.
(86) International application number: JP9702284
(87) International publication number: WO9801536

(57) **Abstract**

Microorganisms belonging to the genus *Shewanella* or *Pseudoalteromonas* which can produce docosahexaenoic acid (DHA) within a short culture period; a process for producing DHA which comprises culturing these microorganisms and isolating a lipid fraction from the culture; and a process for producing DHA labeled with a stable isotope with a high safety by using these microorganisms.

## Description

### TECHNICAL FIELD

The present invention relates to microorganisms capable of producing docosahexaenoic acid, particularly phospholipid containing docosahexaenoic acid; processes for producing docosahexaenoic acid, particularly phospholipid containing docosahexaenoic acid; and a process for producing ¹³C-labeled docosahbexaenoic acid or esters thereof.

### BACKGROUND ART

Derivatives of docosahexaenoic acid (hereinafter referred to as "DHA") include phosphplipids, as well as triglycerides, ethyl esters, amides, and the like. Among these, it has bean found that DHA-containing phospholipid has a more excellent blood lipid improving effect of reducing cholesterol and triglyceride in blood and not reducing high density lipoprotein cholesterol than DHA triglycerides. Additionally, liposomes prepared from phospholipid containing DHA in a large amount have certain characteristics which are not found in liposomes prepared from egg yolk or soybean phospholipid (JP-A-7-68157), so that the DHA-containing phospholipid is useful, for example, as a material of liposomes to be used in the drug delivery system, and the clinical application thereof is expected.

Preparation of the DHA-containing phospholipid has been attempted by processes, such as chemical synthesis {JP-A-51-91213, JP-A-52-89622, JP-A-61-129191 (*Chem. Abs.,* 107:218004d)}, enzymatic ester exchange {*J. Am. Oil Chem. Soc*., 68, 848-851 (1991), *Bulletin of The Japanese Society of Scientific Fisheries*, 59, 309 (1993)}, incorporation into bacterial phospholipid {*J. Am. Oil Chem. Soc*., 71, 325-330 (1994)}, and the like. However, these processes have certain disadvantages in that the DHA content is low, odor of DHA added to the medium remains, and production in a large scale is difficult.

Search for natural sources of the DHA-containing phospholipid has also been attempted, and the extraction from aquatic animals has been reported {JP-A-1-131189 (*Chem. Abs*., 112:11903d), JP-A-7-68157}, but there is a problem in terms of stable production in a large scale. It has been also reported that certain species of marine microalgae {*Phytochemistry*, 30, 2963-2967 (1991), JP-A-7-8221, JP-A-7-95875} and *Mastigomycotina* {*J*. Am. *Oil Chem. Soc*., 68, 509-514 (1991), JP-A-1-199588 (*Chem. Abs*., 112:6133y)} produce DHA, but each of them produces it as DHA-containing triglyceride. Additionally, there are reports on DHA-producing deep-sea microorganisms [*Appl*. *Environ. Microbiol*., 51, 730-737 (1986) {*Vibrio marinus* (ATCC 15381)}; *Lipids*, 29, 527-528 (1994)], but each of the strains requires its culturing under a high pressure or at a low temperature of 5°C or less even under ordinary pressure so that the DHA productivity is low.

In the production method of labeled DHA, DHA labeled with a stable isotope ¹³C is not at all known. Up to the present, DHA labeled with a radioactive isotope ¹⁴C at the 1-position is commercially available, but not only this labeled DHA cannot be used as a tracer of metabolic pathway including β oxidation decomposition process, but also it has many problems with regard to the application to living bodies, especially to human.

### DISCLOSURE OF THE INVENTION

The inventors of the present invention have conducted intensive studies on the screening of microorganisms capable of producing DHA from samples of the sea and found as a result of the efforts that a microorganism belonging to the genus *Shewanella or Pseudoalteromonas* can produce DHA, particularly DHA-containing phospholipid, under ordinary pressure at a low temperature; and that ¹³C-labeled DHA, particularly uniformly ¹³C-labeled DHA, can be obtained efficiently when a ¹³C-labeled carbon source is added to a medium at the time of culturing of the microorganism. The present invention has been accomplished on the basis of these findings.

That is, the present invention relates to microorganisms belonging to the genus *Shewanella* or *Pseudoalteromonas* which can produce DHA, particularly DHA-containing phospholipid; processes for producing DHA, particularly DHA-containing phospholipid, which comprises culturing the microorganism, and isolating a lipid fraction from the obtained culture; and a process for producing ¹³C-labeled DHA or esters thereof. Also, the term "phospholipid containing DHA (DHA-containing phospholipid)" as used herein means phospholipid containing DHA as a constitutive fatty acid.

### BEST MODE FOR CARRYING OUT THE INVENTION

### (1) Microorganisms

The microorganisms of the present invention are not particularly limited so long as they belong to the genus *Shewanella or Pseudoalteromonas* and can produce DHA, particularly DHA-containing phospholipid, and such microorganisms can be isolated from the natural resources, or the mutant strains thereof can be used.

A new strain *Shewanella* sp. SCRC-21406 can be exemplified as the microorganism of the present invention belonging to the genus *Shewanella*. This new strain was deposited in National Institute of Bioscience and Human-technology, Agency of Industrial Science and Technology, as FERM P-15693 (SCRC-21406), and was transferred to international deposition under Budapest Treaty as FERM BP-5979. Also, a new strain *Pseudoalteromonas* sp. SCRC-21416 can be exemplified as the microorganism of the present invention belonging to the genus *Pseudoalteromonas*. This new strain was deposited in National Institute of Bioscience and Human-Technology, Agency of Industrial Science and Technology, as FERM P-15935 (SCRC-21416), and was transferred to international deposition under Budapest Treaty as FERM BP-5980.

The above-described new strains were isolated as set forth below.

**Table 1**

| Medium composition | |
|---|---|
| Peptone | 0.5% |
| Yeast extract | 0.1% |
| Agar | 1.5% |
| Artificial sea water of 1/2 strength | pH 7.0 |

Each of aquatic biological samples collected at various parts of the sea was appropriately diluted with sterilized artificial sea water of 1/2 strength, inoculated onto an agar plate medium of this composition, and cultured at 8°C or less for 3 to 5 days. Each of the thus formed colonies was inoculated into a liquid medium prepared by eliminating agar from the medium composition in Table 1 and allowed to stand for culturing at 8°C or less. The DHA productivity was confirmed with the thus obtained culture broth by a method described below. The following strains capable of considerably producing DHA were obtained in this manner. These samples were collected at the coast of Toyama Bay, Toyama Prefecture, Japan.

The strain *Shewanella* sp*.* SCRC-21406 has the bacteriological properties shown in Table 2 below.

**Table 2**

| SCRC-21406 Items observed I. Morphology |
|---|
| 1. Shape of cell: rod, size 1×3 µm |
| 2. Polymorphism: no |
| 3. Motility: yes, adhesion condition of fragellum: one, polar flagellum |
| 4. Spore: no |
| 5. Gram staining: negative |

| II. Growth on medium |
|---|
| 1. Lab M nutrient medium |
| a) Condition of colony (diameter): 2 mm |
| b) Shape of colony: circular |
| c) Condition of colony surface: smooth |
| d) Raised state of colony: trapezoid |
| e) Periphery of colony: entire |
| f) Color of colony: light yellow |
| g) Transparency of colony: translucent |
| h) Gloss of colony: sharp |

| III. Physiological properties |
|---|
| 1. VP: - |
| 2. MR: + |
| 3. Indole formation: - |
| 4. Hydrogen sulfide formation: - |
| 5. β-Galactosidase: - |
| 6. Citrate utilization : - |
| 7. Gluconate utilisation: + |
| 8. Urease: - |
| 9. Oxidase: + |
| 10. Catalase: + |
| 11. Growth range: a) pH: 6-10, b) temperature: 1°C-20°C |
| 12. Behavior against oxygen: aerobic |
| 13. OF test: (+) |

| 14. Formation of acid and gas from saccharides: (no gas formation) |
|---|
| a) L(+)-arabinose: - |
| b) glucose: + |
| c) inositol: - |
| d) sorbitol: - |
| e) rhamnose: - |
| f) sucrose: - |
| g) melibiose: - |
| h) mannitol: - |

| 15. Other properties |
|---|
| DNase: - |
| Decomposition of ornitine: - |
| Decomposition of arginine: - |
| Decomposition of gelatin: - |
| Hydrolysis of elastin: - |
| Ampicillin sensitivity (10 µg): - |
| Cefalotin sensitivity (30 µg): - |
| Hydrolysis of Tween 80: + |

On the basis of the above bacteriological properties, this strain was identified as follows in accordance with the following references a) to l).

Since the strain SCRC-21406 is aerobic, has motility and one polar flagellum, and is a Gram-negative rod having catalase and oxidase activities, it belongs to the genus *Shewanella* according to reference a). The strain SCRC-21406 is a psychrophilic bacterium because it has a growth temperature of 20°C or less. *Shewanella benthica* is known as a psychrophilic bacterium belonging to the genus *Shewanella*, and its properties described in the reference mostly coincide with the above-described properties, excluding the growth temperature range. Consequently, this strain was identified as a new species analogous to *Shewanella benthica*.

| References | |
|---|---|
| a) | *Bergey's Manual of Systematic Bacteriology*, vol. 1 (1984) |
| b) | *The Prokalyotes* 2Ed., vol. 1-4 (1992) |
| c) | *Cowan and Steel's Manual for the Identification of Medical Bacteria* 3Ed. (1993) |
| d) | *Manual of Non-fermenting Gram-negative Bacteria* (1985) |
| e) | *Identification Method in Applied and Environmental Microbiology* (1992) |
| f) | *Identification Method for Microbiologists* 2nd ed. (1979) |
| g) | *Edwards and Ewings Identification of Enterobacteriaceae* 4Ed. (1986) |
| h) | *The Genus Bacillus* (1973) |
| i) | *The Aerobic Endospore-forming Bacteria* (1981) |
| j) | *SAB Technical Series 14*, 2Ed. (1979) |
| k) | *Oxoid Manual* 5Ed. (1982) |
| l*)* | *Manual of Clinical Microbiology* 6Ed. (1995) |

The strain *Pseudoalteromonas* sp. SCRC-21416 has the bacteriological properties shown in Table 3 below.

**Table 3**

| SCRC-21416 Items observed I. Morphology |
|---|
| 1. Shape of cell: rod, size 1×3 µm |
| 2. Polymorphism : no |
| 3. Motility: yes, adhesion condition of fragellum: one, |
| polar flagellum |
| 4. Spore: no |
| 5. Gram staining: negative |

| II. Growth on medium |
|---|
| 1. Lab M nutrient medium |
| a) Condition of colony (diameter): 2 mm |
| b) Shape of colony: circular |
| c) Condition of colony surface: smooth |
| d) Raised state of colony: raised |
| e) Periphery of colony: entire |
| f) Color of colony: light yellow |
| g) Transparency of colony: translucent |
| h) Gloss of colour: sharp |

| III. Physiological properties |
|---|
| 1. Reduction of nitrate: + |
| 2. Indole formation: - |
| 3. Hydrolysis of starch: + |
| 4. Urease: - |
| 5. Oxidase: + |
| 6. Catalase: + |
| 7. Growth range: a) temperature: 1°C-18°C |
| 8. Behavior against oxygen: aerobic |
| 9. OF test: (+) |
| 10. Formation of acid and gas from glucose (no gas |
| formation): + |

| 11. Assimilation |
|---|
| a) arabinose: - |
| b) glucose: - |
| c) mannose: - |
| d) maltose: - |
| e) mannitol: - |
| f) N-acetylglucosamine: - |
| g) gluconic acid: - |
| h) citric acid : - |
| i) malonic acid: - |
| j) malic acid : - |
| k) capric acid : - |
| l) adipic acid : - |

| 15. Other properties |
|---|
| DNase: + |
| Decomposition of ornitine: - |
| Decomposition of arginine: - |
| Decomposition of gelatin: - |
| Cefalotin sensitivity (30 µg): + |
| Ampicillin sensitivity (10 µg): - |

On the basis of the above bacteriological properties, this strain was identified as follows in accordance with the following references m) to p).

The strain SCRC-21416 is aerobic, has motility and one polar flagellum, and is a Gram-negative rod having catalase and oxidase activities, but there is no known species whose properties completely coincide with these results, including assimilation of saccharides and the like.

However, there are many species in the genus *Pseudoalteromonas* whose biochemical properties are close to those of the strain SCRC-21416. Consequently, this strain was identified as a new species belonging to the genus *Pseudoalteromonas.*

| References | |
|---|---|
| m*)* | *The Prokaryotes* 2Ed., vol. 1-4 (1992) |
| n) | *Bergey's Manual of Determinative Bacteriology,* 9Ed. (1994) |
| o) | *Phylogenetic Analysis of the Genus Alteromonas, Shewanella and Mortierella* (1995) |
| p) | *Int. J. Syst. Bacter*., 45, 755-761 (1995) |

Thus, although the new species isolated from natural resources have been described in detail, strains hawing more high productivity can be obtained by mutating theme new species.

The strains of the present invention can be preserved in the conventional way, for example on an agar slant medium or by a freeze drying method or a glycerol method. With regard to the agar slant medium, the medium described in the foregoing in relation to the isolation of bacteria may be used. The freeze dry preservation and glycerol preservation can also be carried out in the conventional way.

### (2) Production method of DHA

When DHA, particularly DHA-containing phospholipid, is produced by culturing the above-described microorganisms, any medium can be used as the basal nutrient medium so long as the microorganisms of the present invention can grow therein. The medium contains nitrogen sources, such as one or a plurality of yeast extract, peptone, meat extract, and the like. As occasion demands, various saccharides can be added to the medium as carbon sources. It is preferred to add natural sea water or artificial sea water to this medium. The microorganisms may be cultured by using either a solid medium or a liquid medium. However, in order to obtain a large amount of objective DHA, particularly DHA-containing phospholipid, the microorganisms are preferably cultured using a liquid medium under aerobic conditions, much as standing culture, shaking culture, aeration agitation culture, or the like. The culturing temperature is not particularly limited so long as it is within such a range that the microorganisms can grow and produce DHA, and is generally 4 to 20°C, preferably 4 to 15°C. The pH is 6 to 9, preferably 7 to 8. The culturing time is decided by selecting a period by which a collectable amount of the DHA-containing lipid is produced, and is generally 10 to 72 hours.

Next, DHA is collected from the thus obtained culture. A conventionally used lipid purification method can be used as the purification method. For example, cells are collected from the culture broth by conventional methods, such as centrifugation, filtration, and the like. The cells are washed with water, brine, a buffer, such as a phosphate buffer or the like, as occasion demands, and then resuspended in much a liquid. The suspension is extracted with a solvent conventionally used for the extraction of lipid, such as a chloroform/methanol mixture, and then the chloroform phase is obtained by phase separation. Next, the chloroform phase is evaporated to obtain a material containing the DHA-containing phospholipid. Free DHA or a salt thereof can be obtained by saponifying the thus obtained DHA-containing phospholipid in the conventional way, and DHA esters can be obtained by esterification.

### (3) Process for producing ¹³C-labeled DHA

Examples of the microorganism used in the process for producing ¹³C-labeled DHA include microorganisms belonging to the genus *Shewanella* or *Pseudoalteromonas* which produces DHA discovered by the present inventors, such as the new strain SCRC-21406 (FERM BP-5979) which is analogous to *Shewanella benthica*, the new strain SCRC-21416 (FERM BP-5980) belonging to the genus *Pseudoalteromonas*, and *Vibrio marinus* (ATCC 15381). The new strains have been applied to international deposition under Budapest Treaty as described above, and ATCC 15381 can be obtained from ATCC (American Type Culture Collection).

In growing theme microorganisms, any carbon source can be used as the ¹³C-labeled carbon source so long am it can be assimilated, and, for example, sodium acetate can be exemplified.

As an example of the medium to be used in the culturing, a ¹³C-labeled carbon source is added in an amount of 0.05 to 1.0% to a medium composed of 1% of peptone, 0.2% of yeast extract and 50% of artificial sea water. Higher concentration of the ¹³C-labeled carbon source based on carbon sources of the medium results in higher ¹³C content of the DHA produced.

The fatty acid composition of all fatty acid derivatives in the cells can be determined by freeze-drying the cultured cells, carrying out methyl esterification or ethyl esterification with hydrochloric acid-methanol or sodium ethylate according to the conventional method, and then analyzing by GC-MS. Also, the constitutive fatty acid composition of each lipid can be analysed in the same manner after extracting lipids from wet cells or dry cells using an appropriate organic solvent and fractionating the thus extracted lipids by silica gel TLC. When analysed by the above-described method, the cellular fatty acid composition of the present invention was almost the same as that of cells cultured using a conventional medium containing no ¹³C-labeled carbon source. The thus obtained ester of the ¹³C-labeled DHA can be converted into a free form in the conventional way by saponification and subsequent acidification. Also, the term "esters" as used herein include methyl ester, ethyl ester, propyl ester, glyceride, phospholipid, and the like.

The uniformly ¹³C-labeled DHA can be purified by silver nitrate-treated silica gel TLC or silver nitrate-treated silica gel column chromatography, and can be separated from other uniformly ¹³C-labeled fatty acids simultaneously formed.

Next, the present invention is described more specifically by Examples; however, the present invention is not limited thereto.

### EXAMPLES

### Example 1

### Production of DHA-containing phospholipid and DHA methyl ester from Shewanella sp. SCRC-21406:

A 100 ml portion of a medium prepared by dissolving 0.5% of peptone and 0.1% of yeast extract in 1/2 strength of artificial sea water and adjusting a pH of the medium to 7.0 was sterilized at 121°C for 15 minutes, and *Shewanella* sp. SCRC-21406 (FERM BP-5979) was inoculated into the medium and aerobically cultured at 10°C for 30 hours. After culturing, the resulting cells were centrifuged to collect 1.1 g in wet weight of cells. The thus collected cells were washed once with 1/2 strength of artificial sea water, and then suspended in 10 ml of water. The cell suspension was extracted by thoroughly shaking in 40 ml of chloroform/methanol (2:1, v/v), and then centrifuged to obtain the chloroform phase. This extraction step was further repeated twice. The chloroform extraction fractions were concentrated and dried to obtain 5.1 mg of the lipid fraction. When the total lipid was analyzed by silica gel thin layer chromatography, most of the total lipid was phospholipid, and the phospholipid had a composition ratio of phosphatidylethanolamine (PE) to phosphatidylglycerol (PG) of 5:1 (mol/mol). This lipid fraction contained DHA. This fraction was dissolved in a 8% hydrochloric acid methanol solution and heated at 80°C for 1 hour to prepare fatty acid methyl ester. When this fatty acid methyl ester was quantitatively analysed by gas chromatography, it was confirmed that 0.5 mg of DHA methyl ester was contained.

### Reference to the Deposited Microorganism under Regulation 13-2 and the Depository

Depository:
   National Institute of Bioscience and Human-Technology, Agency of Industrial Science and Technology
Address:
   1-3, Higashi 1 chome, Tsukuba-shi, Ibarakiken, Japan
Accession number and date originally deposited:
   June 18, 1996 as FERM P-15693
Accession number and date transferred to international deposition;
   June 13, 1997 as FERM BP-5979

### Example 2

### Production of DHA-containing phospholipid and DHA methyl ester from Shewanella sp. SCRC-21406:

A 100 ml portion of a medium prepared by dissolving 1.0% of peptone and 0.2% of yeast extract in 1/2 strength of artificial sea water and adjusting the medium pH to 7.0 was sterilized at 121°C for 15 minutes, and *Shewanella* sp. SCRC-21406 (FERM BP-5979) was inoculated into the medium and aerobically cultured at 10°C for 39 hours. After culturing, the resulting cells were centrifuged to collect 3.0 g in wet weight of cells. The thus collected cells were washed once with 1/2 strength of artificial sea water, and then suspended in 10 ml of water. The cell suspension was extracted by thoroughly shaking in 40 ml of chloroform/methanol (2:1, v/v), and then centrifuged to obtain the chloroform phase. This extraction step was further repeated twice. The chloroform extraction fractions were concentrated and dried to obtain 6.5 mg of the lipid fraction. This lipid fraction contained DHA. This fraction was dissolved in a 8% hydrochloric acid methanol solution and heated at 80°C for 1 hour to prepare fatty acid methyl ester. When this fatty acid methyl ester was quantitatively analyzed by gas chromatography, it was confirmed that 0.8 mg of DHA methyl ester was contained.

### Example 3

### Production of DHA-containing phospholipid and DHA methyl ester from Shewanella sp. SCRC-21406:

A 100 ml portion of a medium prepared by dissolving 3.0% of peptone and 0.2% of yeast extract in 1/2 strength of artificial sea water and adjusting the medium pH to 7.0 was sterilised at 121°C for 15 minutes, and *Shewanella* sp. SCRC-21406 (FERM BP-5979) was inoculated into the medium and aerobically cultured at 10°C for 39 hours. After culturing, the resulting cells were centrifuged to collect 2.1 g in wet weight of cells. The thus collected cells were washed once with 1/2 strength of artificial sea water, and then suspended in 10 ml of water. The cell suspension was extracted by thoroughly shaking in 40 ml of chloroform/methanol (2:1, v/v), and then centrifuged to obtain the chloroform phase. This extraction step was further repeated twice. The chloroform extraction fractions were concentrated and dried to obtain 10.3 mg of the lipid fraction. This lipid fraction contained DHA. This fraction was dissolved in a 8% hydrochloric acid methanol solution and heated at 80°C for 1 hour to prepare fatty acid methyl ester. When this fatty acid methyl ester was quantitatively analyzed by gas chromatography, it was confirmed that 1.1 mg of DHA methyl ester was contained.

### Example 4

### Production of DHA-containing phospholipid and DHA from Pseudoalteromonas sp. SCRC-21416:

A 100 ml portion of a medium prepared by dissolving 0.5% of peptone and 0.1% of yeast extract in 1/2 strength of artificial sea water and adjusting the medium pH to 7.0 was sterilized at 121°C for 15 minutes, and *Pseudoalteromonas* sp. SCRC-21416 (FERM BP-5980) was inoculated into the medium and aerobically cultured at 8°C for 27 hours. After culturing, the resulting cells were centrifuged to collect 1.1 g in wet weight of cells. The thus collected cells were washed once with 1/2 strength of artificial sea water, and then suspended in 10 ml of water. The cell suspension was extracted by thoroughly shaking in 40 ml of chloroform/methanol (2:1, v/v), and then centrifuged to obtain the chloroform phase. This extraction step was further repeated twice. The chloroform extraction fractions were concentrated and dried to obtain 6.6 mg of the lipid fraction. When the total lipid was analyzed by silica gel thin layer chromatography, most of the total lipid was phospholipid, and the phosphalipid had a composition ratio of phosphatidylethanolamine (PE) to phosphatidylglycerol (PG) of 5:1 (mol/mol). This lipid fraction contained DHA. This fraction was dissolved in a 8% hydrochloric acid methanol solution and heated at 80°C for 1 hour to prepare fatty acid methyl ester. When this fatty acid methyl ester was quantitatively analyzed by gas chromatography, it was confirmed that 0.8 mg of DHA methyl ester was contained.

### Reference to the Deposited Microorganism under Regulation 13-2 and the Depository

Depository:
   National Institute of Bioscience and Human-Technology, Agency of Industrial Science and Technology
Address:
   1-3, Higashi 1 chome, Tsukuba-shi, Ibarakiken, Japan
Accession number and date originally deposited:
   November 7, 1996 as FERM P-15935
Accession number and date transferred to international deposition:
   June 13, 1997 as FERM BP-5980

### Example 5

### Production of DHA-containing phospholipid and DHA from Pseudoalteromonas sp. SCRC-21416:

A 100 ml portion of a medium prepared by dissolving 1.0% of peptone and 0.5% of yeast extract in 1/2 strength of artificial sea water and adjusting the medium pH to 8.0 was sterilized at 121°C for 15 minutes, and *Pseudoalteromonas* sp. SCRC-21416 (FERM BP-5980) was inoculated into the medium and aerobically cultured at 8°C for 44 hours. After culturing, the resulting cells were centrifuged to collect 3.0 g in wet weight of cells. The thus collected cells were washed once with 1/2 strength of artificial sea water, and then suspended in 10 ml of water. The cell suspension was extracted by thoroughly shaking in 40 ml of chloroform/methanol (2:1, v/v), and then centrifuged to obtain the chloroform phase. This extraction step was further repeated twice. The chloroform extraction fractions were concentrated and dried to obtain 13.8 mg of the lipid fraction. This lipid fraction contained DHA. This fraction was dissolved in a 8% hydrochloric acid methanol solution and heated at 80°C for 1 hour to prepare fatty acid methyl ester. When this fatty acid methyl ester was quantitatively analysed by gas chromatography, it was confirmed that 1.4 mg of DHA was contained.

### Example 6

### Production of DHA-containing phospholipid and DHA from Pseudoalteromonas sp. SCRC-21416:

A 100 ml portion of a medium prepared by dissolving 5.0% of peptone and 0.5% of yeast extract in 1/2 strength of artificial sea water and adjusting the medium pH to 7.0 was sterilized at 121°C for 15 minutes, and *Pseudoalteromonas* sp. SCRC-21416 (FERM BP-5980) was inoculated into the medium and aerobically cultured at 4°C for 72 hours. After culturing, the resulting cells were centrifuged to collect 5.95 g in wet weight of cells. The wet cells were extracted by the Bligh-Dyer extraction method {*Can. J. Biochem. Physiol*., 37, 911-917 (1957)}, and the extract was concentrated to dryness to obtain 27.4 mg of the lipid fraction. When this fraction was methyl-esterified, and then quantitatively analysed by gas chromatography, it was confirmed that 4.1 mg of DHA was contained.

### Example 7

A medium was prepared by dissolving 0.3% of [1,2-¹³C] sodium acetate (¹³C: 99 atom %) in P1 medium (1.0% of peptone, 0.2% of yeast extract and 50% of artificial sea water), and the DHA producing strain SCRC-21406 (FERM BP-5979) was inoculated into 100 ml of the thus prepared medium and cultured at 8°C with shaking. The cells were collected at the latter stage of logarithmic growth, and the total lipid was extracted by the Bligh-Dyer extraction method in the conventional way to prepare fatty acid methyl ester. DHA was separated by silver nitrate-treated silica gel TLC and analysed by GC-MS. As the results, while m/z of the parent ion of the DHA methyl ester was 342 when the strain was cultured using the medium to which ¹³C-labeled sodium acetate was not added, ions of 352, 354, 356, 358, 360, 362, 364 and ions having lower ionic strength of 353, 355, 357, 359, 361 and 363 were detected. The ion having the highest strength was 358. In other words, ¹³C-labeled ratio of DHA was from 45 to 100%, and labeled ratio of the ¹³C-labeled DHA having the largest formation ratio was 73%. Thus, 2.9 mg of ¹³C-labeled DHA was obtained. This DHA methyl ester was dissolved in 0.3 N NaOH/90% ethanol, allowed to react for 1 hour at 80°C, acidified with 6 N HCl, and then extracted with n-hexane to obtain a free acid.

### Example 8

A medium was prepared by dissolving 1.0% of [1,2-¹³C] sodium acetate (¹³C: 99 atom %) in P3 medium (3.0% of peptone, 0.2% of yeast extract and 50% of artificial sea water), and the DHA-producing microorganism, SCRC-21406 (FERM BP-5979), was inoculated into 100 ml of the thus prepared medium and cultured at 8°C with shaking. The cells were collected at the latter stage of logarithmic growth, and the total lipid was extracted by the Bligh-Dyer extraction method in the conventional way to prepare fatty acid methyl ester. DHA was separated by silver nitrate-treated silica gel TLC and analysed by GC-MS. As the results, while m/z of the parent ion of the DHA methyl ester was 342 when the strain was cultured using the medium to which ¹³C-labeled sodium acetate was not added, ions of 356, 358, 360, 362, 364 and ions having lover tonic strength of 355, 357, 359, 361 and 363 were detected. The ion having the highest strength was 360. In other words, ¹³C-labeled ratio of DHA was from 64 to 100%, and labeled ratio of the ¹³C-labeled DHA having the largest formation ratio was 82%. Thus, 1.6 mg of ¹³C-labeled DHA was obtained. This DHA methyl ester was dissolved in 0.3 N NaOH/90% ethanol, allowed to react for 1 hour at 80°C, acidified with 6 N HCl, and then extracted with n-hexane to obtain a free acid.

### INDUSTRIAL APPLICABILITY

Docosahexaenoic acid, particularly phospholipid containing the same can be produced by fermentation using the microorganisms of the present invention. According to the processes of the present invention, a large amount of DHA-containing phospholipid, as well as other derivatives of DHA, can be obtained with ease purification and within a short culture period by culturing a microorganism belonging to the genus *Shewanella* or *Pseudoalteromonas*, and isolating the lipid fraction and phospholipid from the culture. The thus obtained DHA can be used as a medicament, a drug carrier, a food additive, a healthy food product, and the like.

Furthermore, DHA labeled with a safe stable isotope is provided by the present invention. By using this labeled DHA, a broad range of metabolic experiments and measurement of metabolic abilities including *in vivo* experiments in human become possible. Because of this, mechanism and the like of useful physiological action mechanism of DHA so far unknown can be revealed, and pharmacological actions of DHA can be utilized more effectively.

## Claims

1. A microorganism belonging to the genus *Shewanella* which can produce docosahexaenoic acid.

2. The microorganism according to claim 1, which is a microorganism analogous to *Shewanella benthica.*

3. The microorganism according to claim 1 or 2, which is *Shewanella* sp. SCRC-21406.

4. A process for producing docosahexaenoic acid, which comprises culturing a microorganism belonging to the genus *Shewanella* which can produce docosahexaenoic acid, and isolating a lipid fraction from the obtained culture.

5. A microorganism belonging to the genus *Pseudoalteromonas* which can produce docosahexaenoic acid.

6. The microorganism according to claim 1, which is *Pseudoalteromonas* sp. SCRC-21416.

7. A process for producing docosahexaenoic acid, which comprises culturing a microorganism belonging to the genus *Pseudoalteromonas* which can produce docosahexaenoic acid and, isolating a lipid fraction from the obtained culture.

8. A process for producing ¹³C-labeled docouahexaenoic acid or esters thereof, which comprises culturing a docosahexaenoic acid-producing microorganism belonging to the genus *Shewanella* or *Pseudoalteromonas* in the presence of a ¹³C-labeled carbon source.

9. ¹³C-Labeled docosahexaenoic acid or esters thereof.
